# EUROPÄISCHE PATENTANMELDUNG

(11) **EP 1 980 929 A1**
(43) Veröffentlichungstag der Anmeldung: **15.10.2008**
(21) Anmeldenummer: 07007473.7
(22) Anmeldetag: 12.04.2007
(51) Int. Cl.: G06F 1/16, A45F 5/02, A61M 5/142

(54) **Schutzhülle für elektronische Kleingeräte**

(71) Anmelder: F.HOFFMANN-LA ROCHE AG, 4070 Basel (CH); Roche Diagnostics GmbH, 68305 Mannheim (DE)
(72) Erfinder: Jeanbourquin, Edgar, 3075 Rüfenacht (CH)
(74) Vertreter: Schalch, Rainer

(57) **Zusammenfassung**

Die Erfindung betrifft eine brillenetuiähnliche Schutzhülle (1) für eine Insulinpumpe, mit zwei steifen Schutzhüllenhälften (13, 14), welche über ein Scharnier (5) derartig miteinander verbunden sind, dass sie im geschlossenen Zustand eine im Wesentlichen steife Schutzhüllenstruktur bilden. Drei der Umfangs- bzw. Kantenbereiche (18), sowie sämtliche Eckbereiche (17), sind aussen von einem Elastomermaterial gebildet.

Eine derartig ausgebildete erfindungsmässige Schutzhülle (1) bietet einen hervorragenden Schutz des Kleingerätes gegen die hohe Stossbeanspruchung, welche typischerweise beim Auftreffen mit einer Ecke (17) auf eine Oberfläche auftreten.

## Beschreibung

Die vorliegende Erfindung betrifft eine Schutzhülle für elektronische Kleingeräte, ein Set umfassend ein elektronisches Kleingerät und eine solche Schutzhülle sowie eine Verwendung der Schutzhülle gemäss den Oberbegriffen der unabhängigen Patentansprüche.

Elektronische Geräte, die am Körper getragen werden, wie z.B. Insulinpumpen, sind besonderen Belastungen ausgesetzt. Daher werden Schutzhüllen verwendet, die die Geräte wirksam vor Beschädigungen schützen sollen.

Für Insulinpumpen sind verschiedene Arten von Schutzhüllen bekannt. Generell kann zwischen weichen und harten Hüllen unterschieden werden. So sind beispielsweise weiche Hüllen aus Neopren bekannt, die wie eine Tasche ausgeformt sind. Die Insulinpumpe wird in die Tasche gesteckt, die dann über eine Lasche verschlossen wird. Aussparungen in der Schutzhülle ermöglichen das Bedienen von Tasten oder anderen Bedienungselementen und das Betrachten des Displays. Des Weiteren sind ähnliche Modelle aus Leder bekannt. Der Nachteil einer solchen Schutzhülle besteht darin, dass das weiche Material relativ wenig Schutz gegen Schläge, z.B. beim Herunterfallen oder durch Sturz des Trägers, bietet.

Harte Schutzhüllen gibt es in verschiedenen Ausführungsformen. So gibt es z.B. von der Firma Roche Diagnostics einen Clipcase, im Wesentlichen eine Schale mit zwei gegenüberliegenden offenen Seiten, in die die Pumpe eingeschoben und durch einen oberen und einen unteren gewölbten Rand gehalten wird. Die Struktur des Clipcase ist leicht federnd ausgebildet, so dass das Einlegen und Herausnehmen der Pumpe erleichtert wird. Auf der Hinterseite der Lagervorrichtung ist ein Federclip zur Befestigung an einem Gürtel angebracht.

Des Weiteren kennt man Schutzhüllen aus einem harten Kunststoff. Die Pumpe wird über eine offene Schmalseite in das passgenaue Gehäuse geschoben und z.B. durch einen Clip arretiert. Auf die Bedienelemente, das Display, wie auch den Katheteranschluss kann durch Öffnungen im Gehäuse zugegriffen werden. Solche so genannten Hardcover weisen jedoch den Nachteil auf, dass sie eine geringe Schockabsorptionsfähigkeit besitzen, so dass eine Beschädigung einer darin enthaltenden und von diesem vollständig umschlossenen Insulinpumpe nicht ausgeschlossen werden kann. Zudem sind diese Schutzhüllen oft derart ausgebildet, dass Ecken oder Kanten ungeschützt bleiben.

Es stellt sich daher die Aufgabe, eine Schutzhülle zur Verfügung zu stellen, welche die zuvor erwähnten Nachteile des Standes der Technik nicht aufweist oder zumindest teilweise vermeidet.

Diese Aufgabe wird durch die Schutzhülle und das Set gemäss den unabhängigen Ansprüchen gelöst.

Demgemäss betrifft ein erster Aspekt der vorliegenden Erfindung eine Schutzhülle für elektronische Kleingeräte, die bei bestimmungsgemässer Verwendung eine im Wesentlichen steife Struktur hat. Unter einer "im Wesentlichen steifen Struktur" wird eine Struktur verstanden, die unter den beim bestimmungsgemässen Gebrauch auftretenden Belastungen keine wesentlichen Formveränderungen erfährt. Zumindest die Eckbereiche der Schutzhülle weisen auf der Aussenseite Elastomermaterial auf, derart, dass die Stossbelastung der Schutzhülle beim Auftreffen mit einer Ecke auf eine ebene Oberfläche durch dieses Elastomermaterial gedämpft wird. Eine derartig ausgebildete erfindungsmässige Schutzhülle bietet einen hervorragenden Schutz des Kleingerätes gegen die hohe Stossbeanspruchung, welche typischerweise beim Auftreffen mit einer Ecke auf eine Oberfläche auftreten.

Unter einem anspruchsgemässen "Eckbereich" werden ganz allgemein exponierte Aussenbereich der Schutzhülle verstanden, an denen Aussenflächen der Schutzhülle eckig oder gerundet ineinander übergehen, derart, dass beim Auftreffen dieses Bereichs auf eine ebene Oberfläche eine im Wesentlichen punktförmige Berührung mit der Oberfläche stattfindet.

In einer bevorzugten Ausführungsform befindet sich auf der Aussenseite entlang von Aussenkanten, bevorzugterweise entlang sämtlicher Aussenkanten, Elastomermaterial, derart, dass die Stossbelastung der Schutzhülle beim Auftreffen mit einer Aussenkante auf eine ebene Oberfläche durch dieses Elastomermaterial gedämpft wird. Hierdurch lässt sich ein deutlich erhöhter Schutz für das darin geschützte Kleingerät erzielen.

Unter anspruchsgemässen "Aussenkanten" werden ganz allgemein exponierte Aussenbereich der Schutzhülle verstanden, an denen Aussenflächen der Schutzhülle eckig oder gerundet ineinander übergehen, derart, dass beim Auftreffen dieses Bereichs auf eine ebene Oberfläche eine im Wesentlichen linienförmige Berührung mit der Oberfläche stattfindet.

In einer weiteren bevorzugten Ausführungsform ist auf der Aussenseite der Schutzhülle Elastomermaterial derartig angeordnet, dass die Stossbelastung der Schutzhülle bei einem Auftreffen derselben mit beliebiger Orientierung auf eine ebene Oberfläche durch das Elastomermaterial gedämpft wird. Dadurch wird ein bestmöglicher Schutz des in der Schutzhülle enthaltenen elektronischen Kleingeräts gewährleistet.

In noch einer weiteren bevorzugten Ausführungsform umfasst die steife Schutzhüllenstruktur zwei steife Schutzhüllenhälften mit jeweils mindestens einem starren Strukturelement, welche über ein oder mehrere Scharnierelemente miteinander verbunden sind. Dadurch wird ein Aufklappen der Schutzhülle ermöglicht, und dadurch die Entnahme bzw. die Einbringung des elektronischen Kleingeräts aus der bzw. in die Schutzhülle erleichtert. Ein Scharnierelement kann auf verschiedenste Weise realisiert werden. Zum Beispiel kann ein Scharnierelement mit einer Achse vorhanden sein. Bevorzugterweise sind die Scharnierelemente durch einstückige Verbindungselemente aus Elastomermaterial gebildet. Hierdurch lassen sich einfach und kostengünstig derartige Schutzhüllen bereitstellen, die zudem im Scharnierbereich eine gute Dämpfung ermöglichen.

In noch einer weiteren bevorzugten Ausführungsform ist ein Verschlussmechanismus vorhanden, mittels welchem zwei starre Strukturelemente der Schutzhüllenhälften bei bestimmungsgemässer Verwendung derartig miteinander verbunden oder verbindbar sind, dass die Schutzhülle gegen ein unbeabsichtigtes Sichöffnen gesichert ist. Verschiedene Schnapp-, Klett- oder andere Systeme können als Verschlussmechanismus dienen. Bevorzugterweise ist der Verschlussmechanismus von Formelementen der starren Strukturelemente gebildet, mit Vorteil derart, dass die beiden Strukturelemente der Schutzhülle miteinander verrastbar sind. So ergibt sich ein einfacher, robuster Aufbau.

In noch einer weiteren bevorzugten Ausführungsform weist die Schutzhülle bezogen auf ihre Grundform eine Symmetrieebene auf. Dabei verläuft die Trennebene zwischen den steifen Schutzhüllenhälften bevorzugterweise im Wesentlichen entlang der Symmetrieebene. Dadurch wird insbesondere die Entnahme des Kleingeräts aus der geöffneten Schutzhülle erleichtert.

In noch einer weiteren bevorzugten Ausführungsform weist die Schutzhülle mindestens eine Öffnung auf, durch welche bei bestimmungsgemässer Verwendung ein in dessen Innenraum angeordnetes Kleingerät zugänglich ist. Dabei ist es bei den Ausführungsformen, bei denen die Schutzhüllenstruktur mindestens zwei steife Schutzhüllenhälften aufweist, welche über ein oder mehrere Scharnierelemente miteinander verbunden sind, bevorzugt, dass die Schutzhülle mindestens eine Öffnung aufweist, welche beim Aufklappen derselben umfangsmässig geöffnet wird. Das ermöglicht, Formelemente des aufzunehmenden elektronischen Kleingeräts im geöffneten Zustand der Schutzhülle in radialer Richtung bezogen auf die Öffnung in diese einzubringen und diese durch Schliessen der Schutzhülle radial formschlüssig zu umschliessen.

In noch einer weiteren bevorzugten Ausführungsform weist die erfindungsgemässe Schutzhülle mindestens einen Bereich aus durchsichtigem Material auf, durch welchen bei bestimmungsgemässer Verwendung ein in dessen Innenraum angeordnetes Kleingerät zumindest teilweise sichtbar ist. Dieser Bereich dient beispielsweise als Sichtfenster für ein Display oder zur Überprüfung des Füllstandes einer Insulinampulle.

In noch einer weiteren bevorzugten Ausführungsform weist die Schutzhülle zumindest einen Bereich aus elastischen, z.B. folienartigen, bevorzugterweise durchsichtigem Material auf, durch welchen hindurch druckbedienbare Bedienelemente eines im Innenraum der Schutzhülle angeordneten Kleingeräts bedienbar sind. Dadurch muss das elektronische Gerät bei bestimmungsgemässer Verwendung der Schutzhülle zum Bedienen nicht aus der Hülle genommen werden.

In noch einer weiteren bevorzugten Ausführungsform weist ein starres Strukturelement der Schutzhülle auf seiner Aussenseite Befestigungsmittel zur Befestigung der Schutzhülle an der Bekleidung eines Benutzers auf. Verschiedene Befestigungsmittel und Tragsysteme sind denkbar. Bevorzugterweise sind ein oder mehrere Clips und/oder Schlaufen zur Befestigung an einem Gürtel vorhanden. In einer bevorzugten Ausführungsform befinden sich die Befestigungsmittel auf dem starren Strukturelement einer Schutzhüllenhälfte. Bevorzugterweise ist das Befestigungsmittel austauschbar an dem starren Strukturelement angebracht, so dass der Benutzer je nach Bedarf die Befestigungsmittel austauschen kann.

In noch einer weiteren bevorzugten Ausführungsform umschliesst die Schutzhülle den von ihr gebildeten Innenraum allseitig. Dadurch wird bei bestimmungsgemässer Verwendung ein umfassender Rundumschutz des elektronischen Kleingeräts gewährleistet.

In noch einer weiteren bevorzugten Ausführungsform ist die Schutzhülle einstückig. Dadurch wird die Gefahr vermieden, dass einzelne Komponenten verloren gehen können.

In noch einer weiteren bevorzugten Ausführungsform ist die Schutzhülle wasserfest und bietet so bei bestimmungsgemässer Verwendung Schutz vor Spritzwasser oder Schweiss.

In noch einer weiteren bevorzugten Ausführungsform weist die Schutzhülle zusätzlich zu dem Innenraum für die Aufnahme des Kleingeräts einen Stauraum für Kleinobjekte auf. Auf diese Weise können z.B. eine Ersatzbatterie oder eine Ersatzinsulinampulle mitgeführt werden.

Ein zweiter Aspekt der Erfindung betrifft ein Set umfassend ein elektronisches Kleingerät, bevorzugterweise eine Pumpe zur Förderung eines flüssigen Medikaments, weiter bevorzugt eine Insulinpumpe, und eine darauf angepasste Schutzhülle gemäss dem ersten Aspekt der vorliegenden Erfindung. "Angepasst" bedeutet im vorliegenden Fall, dass der Innenraum der Schutzhülle die Gegenkontur zur Aussenkontur des Kleingerätes bildet, derart, dass dieses im Wesentlichen spielfrei darin aufgenommen werden kann. Hierdurch ergibt sich ein optimaler Schutz für das umfasste Kleingerät.

Ein dritter Aspekt der Erfindung betrifft die Verwendung der Schutzhülle gemäss dem ersten Aspekt der vorliegenden Erfindung, zum Schutz einer Pumpe zur Förderung eines flüssigen Medikaments, bevorzugterweise einer Insulinpumpe. Bei solcher Verwendung treten die Vorteile der Erfindung besonders deutlich zu Tage.

Weitere Vorteile und Anwendungen der Erfindung ergeben sich aus der nun folgenden Beschreibung anhand der Figuren. Dabei zeigen:
Fig. 1 eine perspektivische Vorderansicht auf eine erfindungsgemässe Schutzhülle bei bestimmungsgemässer Verwendung mit einer Insulinpumpe angeordnet in ihrem Innenraum;
Fig. 2a einen Schnitt entlang der Linie A - A in Fig. 2b durch die Schutzhülle jedoch im aufgeklappten Zustand ohne Insulinpumpe;
Fig. 2b eine Seitenansicht auf die Schutzhülle aus Fig. 1;
Fig. 3 eine perspektivische Rückansicht der Schutzhülle aus Fig. 1 mit Basisplatte; und
die Figuren 3a, 3b und 3c drei perspektivische Rückansichten der Schutzhülle mit jeweils verschiedenen Befestigungsmitteln.

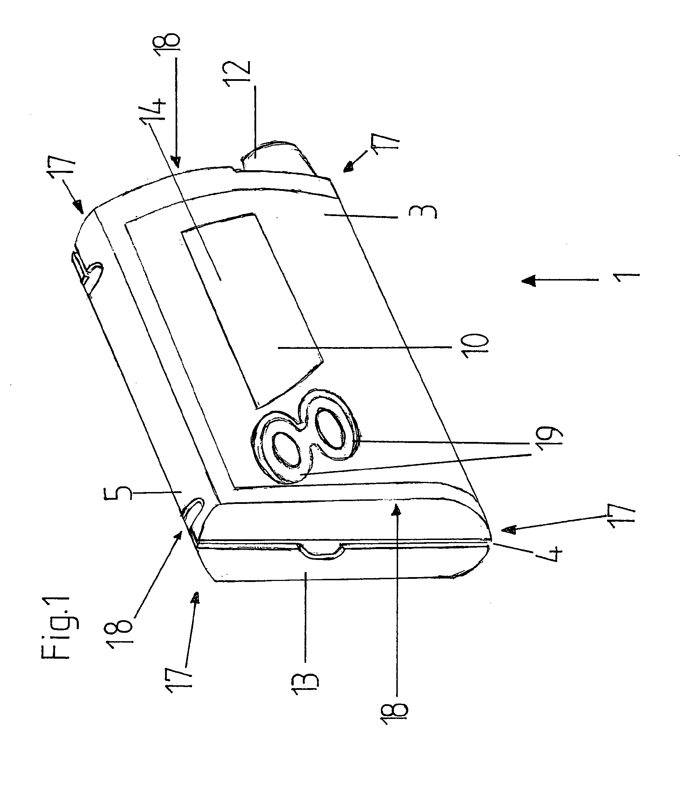

Fig. 1 zeigt eine Vorderansicht einer einteiligen Schutzhülle 1 mit einer in deren Innenraum angeordneten Insulinpumpe. Im dargestellten Zustand, welcher die bestimmungsgemässe Verwendung der Schutzhülle 1 zum Schutz einer Insulinpumpe zeigt, ist diese vollständig geschlossen, wobei sie die Pumpe im Wesentlichen spielfrei umschliesst. Die Schutzhülle 1 hat bezüglich der Grundform einen symmetrischen Aufbau mit einer Symmetrieebene, welche gleichzeitig die Trennebene 4 zwischen zwei Schutzhüllenhälften 13, 14 bildet. Die vordere Hälfte 14 bildet die Vorderseite der Schutzhülle 1 und umfasst ein starres Strukturelement 3, z.B. aus Hart-PVC oder Polyamid, mit einem Sichtfenster 10 zur Sichtbarmachung des Pumpendisplays und zwei elastische Membranen 19 aus Gummi, durch welche hindurch die Bedienelemente der Insulinpumpe bedienbar sind. Drei der Umfangs- bzw. Kantenbereiche 18, sowie sämtliche Eckbereiche 17, sind aus einem Elastomermaterial gebildet, im vorliegenden Fall aus Gummi. Der Anschluss 12 für die Katheterleitung der Insulinpumpe ragt seitlich aus der Schutzhülle 1 hervor.

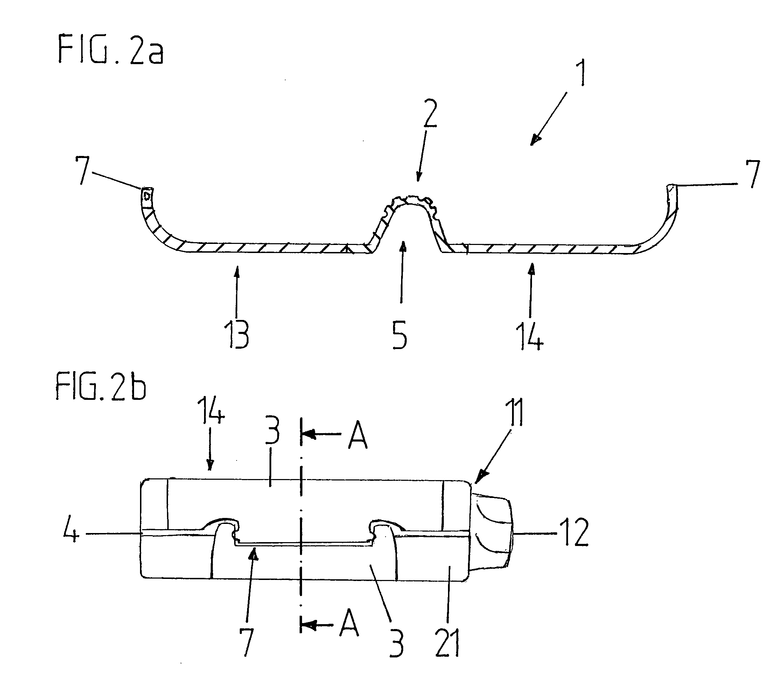

Fig. 2a zeigt einen Schnitt durch die Schutzhülle 1 im offenen Zustand, und zeigt in Zusammenschau mit Fig. 1, dass die beiden Schutzhüllenhälften 13, 14 über ein Elastomerband 5, welches auf der Innenseite Längsrillen 2 aufweist, scharnierartig miteinander verbunden sind, derart, dass die Schutzhülle 1 entlang der Trennlinie 4 vollständig auseinandergeklappt werden kann. Die Längsrillen 2 erhöhen die Schockabsorptionsfähigkeit in diesem Bereich. Wie weiter in Zusammenschau mit Fig. 2b erkennbar ist, welche eine Seitenansicht auf die dem Elastomerband 5 gegenüberliegende Seite der Schutzhülle 1 im geschlossenen Zustand zeigt, sind die beiden Schutzhüllenhälften 13, 14 im geschlossenen Zustand mittels eines rastbaren Verschlussmechanismus 7 miteinander verbunden. Dieser Mechanismus 7 wird jeweils durch Formelemente der starren Strukturelemente der Schutzhüllenhälften 13, 14 gebildet.

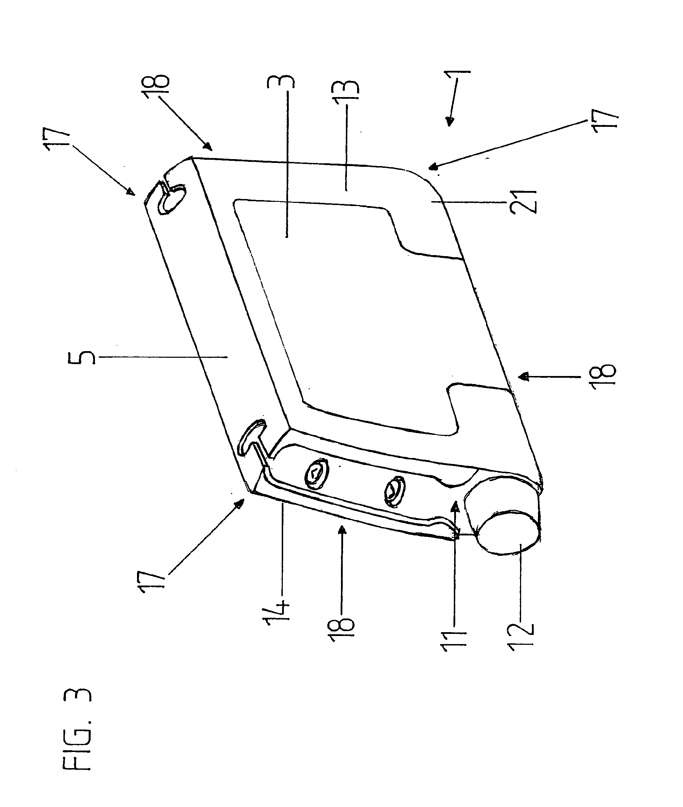

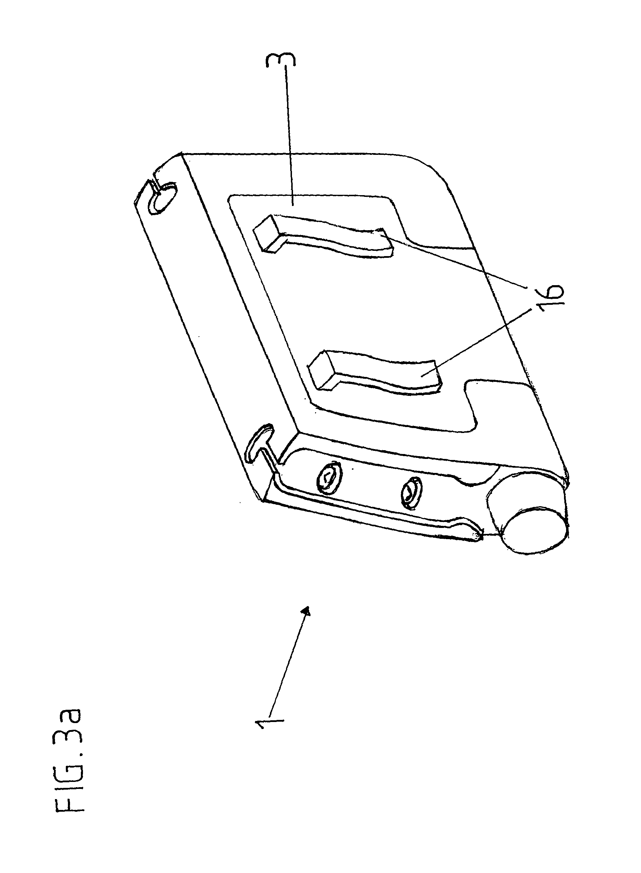

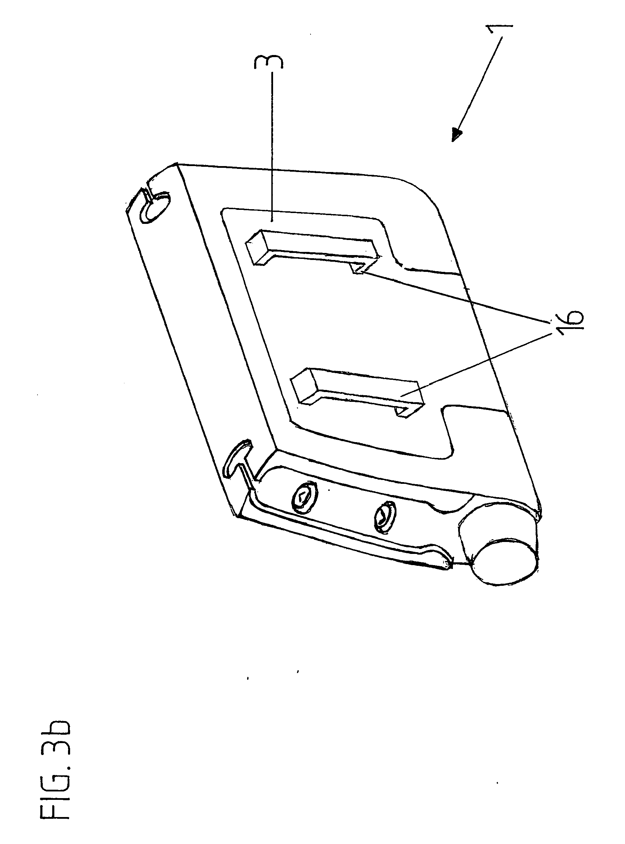

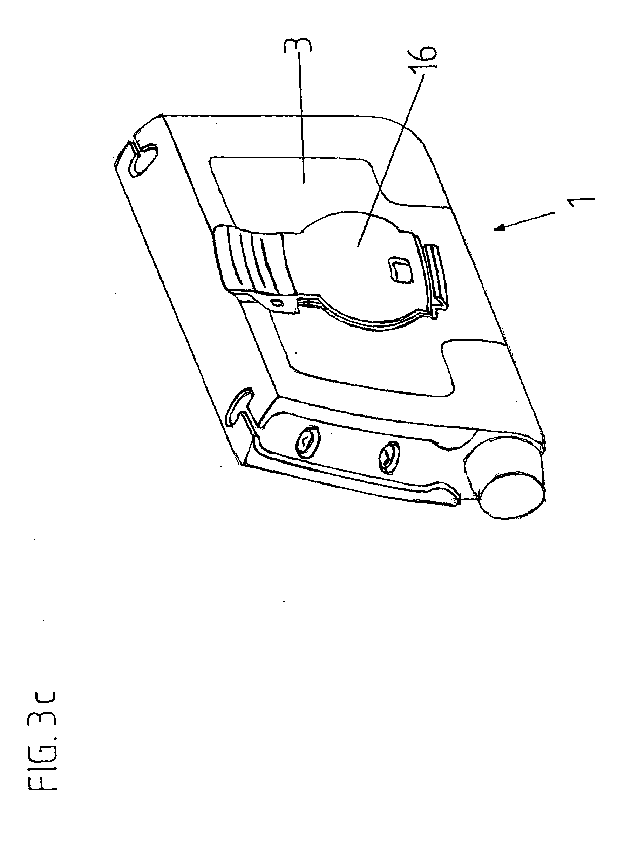

Fig. 3 zeigt eine perspektivische Rückansicht der Schutzhülle 1 im geschlossenen Zustand. Die hier gezeigte hintere Hälfte 13 der Schutzhülle 1 umfasst ebenfalls ein starres Strukturelement 3 aus Hart-PVC, im Weiteren auch als Basisplatte 3 bezeichnet.

Fig. 3a zeigt eine Ausführungsvariante der Basisplatte 3, an der zwei Clips 16 als Befestigungsmittel angeordnet sind. Über die Clips 16 kann die Schutzhülle an der Kleidung des Benutzers befestigt werden.

In Fig. 3b sieht man eine weitere Ausführungsvariante der Basisplatte 3, an der zwei Schlaufen 16 als Befestigungsmittel gebildet sind. Hiermit kann die Schutzhülle 1 an einem Gürtel befestigt werden.

Fig. 3c zeigt eine weitere Ausführungsvariante der Basisplatte 3, an der ein Federclip 16 zur Befestigung an der Kleidung angeordnet ist. Dieser Federclip 16 ist drehbar und erlaubt so unterschiedliche Positionierungen bezüglich der Schutzhülle 1.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Schutzhülle für elektronische Kleingeräte, mit einer bei bestimmungsgemässer Verwendung im wesentlichen steifen Schutzhüllenstruktur, wobei zumindest die Eckbereiche (17) der Schutzhülle (1) auf der Aussenseite Elastomermaterial aufweisen, derart, dass die Stossbelastung der Schutzhülle (1) beim Auftreffen mit einer Ecke (17) auf eine ebene Oberfläche gedämpft wird.

2. Schutzhülle gemäss Anspruch 1, **dadurch gekennzeichnet, dass** die Schutzhülle (1) auf der Aussenseite entlang von Aussenkanten (18), insbesondere entlang sämtlicher Aussenkanten (18), Elastomermaterial aufweist, derart, dass die Stossbelastung der Schutzhülle (1) beim Auftreffen mit einer Aussenkante auf eine ebene Oberfläche gedämpft wird.

3. Schutzhülle gemäss einem der vorangehenden Ansprüche, **dadurch gekennzeichnet, dass** auf der Aussenseite der Schutzhülle (1) Elastomermaterial derartig angeordnet ist, dass die Stossbelastung der Schutzhülle (1) bei einem Auftreffen derselben mit beliebiger Orientierung auf eine ebene Oberfläche gedämpft wird.

4. Schutzhülle gemäss einem der vorangehenden Ansprüche, **dadurch gekennzeichnet, dass** die steife Schutzhüllenstruktur zwei steife Strukturhüllenhälften (13,14) mit jeweils mindestens einem starren Strukturelement (3) umfasst, welche über ein oder mehrere Scharnierelemente (5) miteinander verbunden sind, zur Ermöglichung eines Aufklappens der Schutzhülle (1) zwecks Entnahme bzw. Einbringung des elektronischen Kleingeräts.

5. Schutzhülle gemäss Anspruch 4, **dadurch gekennzeichnet, dass** die Scharnierelemente (5) durch insbesondere einstückige Verbindungselemente aus Elastomermaterial gebildet sind.

6. Schutzhülle gemäss einem der Ansprüche 4 bis 5, **dadurch gekennzeichnet, dass** ein Verschlussmechanismus (7) vorhanden ist, mittels welchem zwei starre Strukturelemente (3) der Schutzhüllenhälften (13, 14) bei bestimmungsgemässer Verwendung derartig miteinander verbunden oder verbindbar sind, dass die Schutzhülle (1) gegen ein unbeabsichtigtes Sichöffnen gesichert ist.

7. Schutzhülle gemäss Anspruch 6, **dadurch gekennzeichnet, dass** der Verschlussmechanismus (7) von Formelementen der starren Strukturelemente (3) gebildet ist, insbesondere derart, dass die beiden Strukturelemente (3) miteinander verrastbar sind.

8. Schutzhülle gemäss einem der Ansprüche 4 bis 7, **dadurch gekennzeichnet, dass** die Schutzhülle (1) bezogen auf ihre Grundform eine Symmetrieebene aufweist.

9. Schutzhülle gemäss Anspruch 8, **dadurch gekennzeichnet, dass** die Trennebene (4) zwischen den steifen Schutzhüllenhälften (13, 14) im Wesentlichen entlang der Symmetrieebene verläuft.

10. Schutzhülle gemäss einem der vorangehenden Ansprüche, **dadurch gekennzeichnet, dass** die Schutzhülle (1) mindestens eine Öffnung (11) aufweist, durch welche bei bestimmungsgemässer Verwendung ein in dessen Innenraum angeordnetes Kleingerät zugänglich ist.

11. Schutzhülle gemäss Anspruch 4 und Anspruch 10, **dadurch gekennzeichnet, dass** die Schutzhülle (1) mindestens eine Öffnung (11) aufweist, welche beim Aufklappen der Schutzhülle (1) umfangsmässig geöffnet wird.

12. Schutzhülle gemäss einem der vorangehenden Ansprüche, **dadurch gekennzeichnet, dass** die Schutzhülle (1) mindestens einen Bereich (10) aus durchsichtigem Material aufweist, durch welchen bei bestimmungsgemässer Verwendung ein in dessen Innenraum angeordnetes Kleingerät zumindest teilweise sichtbar ist.

13. Schutzhülle gemäss einem der vorangehenden Ansprüche, **dadurch gekennzeichnet, dass** zumindest ein Bereich (19) aus einem elastischen, insbesondere folienartigen, insbesondere durchsichtigem Material vorhanden ist, durch welchen hindurch druckbedienbare Bedienelemente eines im Innenraum der Schutzhülle (1) angeordneten Kleingeräts bedienbar sind.

14. Schutzhülle gemäss einem der vorangehenden Ansprüche, **dadurch gekennzeichnet, dass** ein starres Strukturelement (3) der Schutzhülle (1) auf seiner Aussenseite Befestigungsmittel (16) zur Befestigung der Schutzhülle (1) an der Bekleidung eines Benutzers, insbesondere einen oder mehrere Clips und/oder Schlaufen zur Befestigung an einem Gürtel, aufweist.

15. Schutzhülle gemäss Anspruch 14, **dadurch gekennzeichnet, dass** die Befestigungsmittel (16) austauschbar am starren Strukturelement (3) angebracht sind.

16. Schutzhülle gemäss einem der vorangehenden Ansprüche, **dadurch gekennzeichnet, dass** die Schutzhülle (1) den von ihr gebildeten Innenraum allseitig umschliesst.

17. Schutzhülle gemäss einem der vorhergehenden Ansprüche, **dadurch gekennzeichnet, dass** die Schutzhülle (1) einstückig ist.

18. Schutzhülle gemäss einem der vorhergehenden Ansprüche, **dadurch gekennzeichnet, dass** die Schutzhülle (1) wasserfest ist.

19. Schutzhülle gemäss einem der vorhergehenden Ansprüche, **dadurch gekennzeichnet, dass** die Schutzhülle (1) zusätzlich zu dem Innenraum für die Aufnahme des Kleingeräts einen Stauraum für Kleinobjekte aufweist.

20. Set umfassend ein elektronisches Kleingerät, insbesondere eine Pumpe zur Förderung eines flüssigen Medikaments, insbesondere eine Insulinpumpe, und eine darauf angepasste Schutzhülle (1) gemäss einem der vorangehenden Ansprüche.

21. Verwendung der Schutzhülle (1) gemäss einem der Ansprüche 1 bis 19 zum Schutz einer Pumpe zur Förderung eines flüssigen Medikaments, insbesondere einer Insulinpumpe.
